# EUROPEAN PATENT APPLICATION

(11) **EP 1 810 697 A2**
(43) Date of publication of application: **25.07.2007**
(21) Application number: 06123557.8
(22) Date of filing: 07.11.2006
(51) Int. Cl.: A61L 15/00

(54) **Devices for the delivery of molecular sieve materials for the formation of blood clots**

(30) Priority: 07.11.2005 US 734392 P
(71) Applicant: Horn, Jeffrey L., Rocky Hill, CT 06067 (US); Huey, Raymond J., Orange, CT 06477 (US)
(72) Inventor: Horn, Jeffrey L., Rocky Hill, CT 06067 (US); Huey, Raymond J., Orange, CT 06477 (US)
(74) Representative: Israelsson, Stefan

(57) **Abstract**

An apparatus for promoting the clotting of blood comprises a substrate and a zeolite in powder form deposited on the substrate. At least a portion of the substrate is selected from a group consisting of paper, polymer matrix, polyethylene sheet, hydrophilic macromolecules, and cloth. An agent for promoting the clotting of blood comprises a porous web structure and a compound capable of providing hemostasis incorporated into the porous web structure. The compound capable of providing hemostasis comprises at least one cationic species interspersed throughout and coulombically bound to the web structure such that the cationic species provides a positive charge to the web structure. A method of fabricating a blood clotting apparatus comprises the steps of providing a cellulose-based substrate; providing a zeolite; incorporating at least one cationic species into a structure of the zeolite to impart a positive charge thereto; and impregnating the cellulose-based substrate with the zeolite.

## Description

### CROSS REFERENCE TO RELATED APPLICATION

This application claims the benefit of U.S. Provisional Patent Application No. 60/734,392, filed on November 7, 2005, the contents of which are incorporated herein by reference in their entirety.

### TECHNICAL FIELD

The present invention relates generally to blood clotting devices and, more particularly, to blood clotting materials, devices incorporating such materials and methods for the delivery of such materials for use in controlling bleeding.

### BACKGROUND OF THE INVENTION

Controlling bleeding is often the most critical concern when traumatic injuries occur or surgical procedures are undertaken. The location of the bleed site can make controlling bleeding easier or considerably more difficult. In mammals, certain of the internal organs do not heal properly when either traumatized by impact or after surgical procedures to repair or remove portions of those organs. The liver and spleen are particularly prone to low recovery rates after damage and repair involving large scale bleeding. The kidney and brain tissue are also problematic regarding full recovery after trauma involving large scale bleeding episodes through the organ walls. Successful conclusion of the surgical procedure requires that the bleeding be substantially stopped prior to closing of the surgical site. Available methods of stopping bleeding all have drawbacks and limitations and improved methods of assisting natural clotting are needed.

Blood is a liquid tissue that includes red cells, white cells, corpuscles, and platelets dispersed in a liquid phase. The liquid phase is plasma, which includes acids, lipids, solubilized electrolytes, and proteins. The proteins are suspended in the liquid phase and can be separated out of the liquid phase by any of a variety of methods such as filtration, centrifugation, electrophoresis, and immunochemical techniques. One particular protein suspended in the liquid phase is fibrinogen. When bleeding occurs, the fibrinogen reacts with water and thrombin (an enzyme) to form fibrin, which is insoluble in blood and polymerizes to form clots.

In a wide variety of circumstances, animals, including humans, can be wounded. Often bleeding is associated with such wounds. In some circumstances, the wound and the bleeding are minor, and normal blood clotting functions in addition to the application of simple first aid are all that is required. Unfortunately, however, in other circumstances substantial bleeding can occur. To stop or reduce bleeding in these situations generally requires specialized equipment and materials as well as personnel trained to administer appropriate aid. If such aid is not readily available, excessive blood loss can occur. When bleeding is severe, sometimes the immediate availability of equipment and trained personnel is still insufficient to stanch the flow of blood in a timely manner and the person or animal can bleed to death

Moreover, severe wounds can often be inflicted in remote areas or in situations, such as on a battlefield, where adequate medical assistance is not immediately available. In these instances, it is important to stop bleeding, even in less severe wounds, long enough to allow the injured person or animal to receive medical attention

In an effort to address the above-described problems, materials have been developed for controlling excessive bleeding in situations where conventional aid is unavailable or less than optimally effective. Although these materials have been shown to be somewhat successful, they are sometimes not effective enough for traumatic wounds and tend to be expensive. Furthermore, in some situations these materials are ineffective and/or can be difficult to apply as well as remove from a wound.

In surgical procedures, bleeding is often controlled by suturing or stapling an incision or internally bleeding area. Sponges or other materials can be used to exert pressure against the bleed site and/or absorb the blood. However, when the bleeding becomes excessive, these measures may not be sufficient to stop the flow of blood. Moreover, some organs like the liver or spleen cannot be easily sutured or stapled.

Historically zeolite materials have been used as a blood coagulant however, the material form and composition have been highly exothermic when contact is made with blood. This exothermia can be highly undesirable depending on the nature of the wound.

There is a need for an improved clotting device that can quickly stop bleeding or hemorrhaging from wounds, incisions or significant bruising with bleeding. Regarding surgical procedures, the need for a quick, effective clotting technique in tissue prone to continued bleed-through has been long recognized in both human and veterinary surgery.

Based on the foregoing, it is a general object of the present invention to provide devices for controlling bleeding and methods of their use that overcome or improve upon the prior art.

### SUMMARY OF THE INVENTION

According to one aspect, the present invention resides in an apparatus for promoting the clotting of blood. The apparatus comprises a substrate and a zeolite in powder form deposited on the substrate. The zeolite comprises a matrix of porous aluminosilicate and at least one cationic species interspersed throughout and coulombically bound to the matrix such that a positive charge is imparted to the matrix. At least a portion of the substrate is selected from a group consisting of paper (e.g., adsorbent media paper), polymer matrix in sheet form, polyethylene sheet, hydrophilic macromolecules, natural cloth, synthetic cloth, non-woven natural cloth, and non-woven synthetic cloth.

According to another aspect, the present invention resides in an agent for promoting the clotting of blood. Such an agent comprises a porous web structure and a compound capable of providing hemostasis incorporated into the porous web structure. The compound capable of providing hemostasis comprises at least one cationic species interspersed throughout and coulombically bound to the web structure such that the cationic species provides a positive charge to the web structure.

According to another aspect, the present invention resides in a method of fabricating a blood clotting apparatus. The method comprises the steps of providing a cellulose-based substrate; providing a zeolite; incorporating at least one cationic species into a structure of the zeolite to impart a positive charge thereto; and impregnating the cellulose-based substrate with the zeolite.

Other aspects of the present invention include a pad for controlling bleeding and a bandage applicable to a bleeding wound. The pad and the bandage each employ at least one substrate having particles of zeolite (or other molecular sieve material) retained thereon. The pad can include a substantially impermeable layer of material opposite the portion of the substrate having the zeolite thereon, and a rigid or semi-rigid support can be attached to the substrate to facilitate ease of the handling and the application of pressure to the pad where it is placed on a bleed site.

With regard to a bandage, a flexible web can be attached to the above-described substrate, the flexible web being a cloth or polymeric material. In yet another aspect of the present invention, a method of dressing a bleeding wound includes providing a zeolite (or other molecular sieve material) in particle form and retaining the material on a substrate, placing the substrate on a bleeding wound such that the zeolite comes into contact with blood flowing from the wound, and applying pressure to the substrate to ensure contact of the material with the blood, thereby promoting clothing and removing the substrate from the wound.

In the preferred embodiment of the present invention, the zeolite found to be particularly effective in causing blood to clot while minimizing any exothermia is in powder form and contains less than about 75% by weight silicon oxide, and preferably less than about 65% by weight silicon oxide; less than about 50% by weight aluminum oxide, and preferably less than about 40% by weight of aluminum oxide; less than about 30% by weight sodium oxide, and preferably less than about 20% by weight of sodium oxide; less about 30% by weight of calcium oxide, and preferably less than about 20% by weight of calcium oxide. The zeolite powder is impregnated into the substrate which in the preferred embodiment is a paper. While the material has been described as being impregnated into the paper, the present invention is not limited in this regard as the zeolite can also be adhesively attached to the paper without departing from the broader aspects of the invention.

An advantage of the present invention is that upon completion of the application of any of the devices of the present invention to a bleeding wound, the devices can be easily removed. In particular, because the zeolite material is embedded in or otherwise attached to the substrate, the entire apparatus can be easily and cleanly pulled away from the treated wound and disposed of with little or no zeolite remaining at the bleed site. Accordingly, little or no irrigation of the wound is required to flush away remaining zeolite. In apparatuses in which the substrate containing zeolite material is incorporated into an adhesive bandage, the device can be left on the wound for the amount of time necessary to cause clotting or longer if conditions warrant.

Another advantage is that the powder form of the particular zeolite material utilized allows the material to react less exothermically. The porous nature of the material still allows liquid blood constituents to be wicked away to cause thickening of the blood, thereby facilitating the formation of clots. Because the initial moisture content of the zeolite is controlled in a preferred embodiment, a less aggressive drawing of moisture from the blood is realized, which thereby tempers the exothermic effects experienced at the wound site.

Still another advantage of the present invention is that the proper dose of zeolite can be readily applied to an open wound. Particularly when the device is a porous substrate containing the zeolite material, the device can be readily removed from a sterilized packaging and held directly at the points from which blood emanates to facilitate clotting of the blood without spilling powder or pellets outside the wound area. Guesswork, estimation, or calculation of the amounts of zeolite for application to a bleeding wound is eliminated since there is a definite amount of the zeolite powder embedded in the substrate. Accordingly, little or no zeolite is wasted.

### BRIEF DESCRIPTION OF THE DRAWINGS

FIG. 1 is a schematic representation of a highly magnified section of a blood clotting device of the present invention.
FIG. 2 is a side view of the blood clotting device of FIG. 1 illustrating one means of retaining of molecular sieve powder in a substrate.
FIG. 3 is a side view of a pressure pad incorporating the molecular sieve powder embedded in the substrate for pressure application to a bleeding wound.
FIG. 4 is a perspective view of a bandage incorporating the molecular sieve powder in a substrate for application to a bleeding wound.
FIG. 5 is a cross sectional magnified view of a single pore of an open-cell foam substrate with zeolite particle embedded in the pore
FIG. 6 is a cross sectional magnified view of a portion of a honeycomb cell foam substrate with breaks in cell walls
FIG. 7 is a photomicrograph of a section of a polymeric open cell foam matrix suitable for inclusion of zeolite in the cell structure.

### DETAILED DESCRIPTION OF THE PREFERRED EMBODIMENTS

In an embodiment of the present invention, a porous web is employed to retain a hemostatic (blood clotting) compound therein for application to a bleeding wound. Although the hemostatic compound is referred to as being a molecular sieve material in fine particulate or powder form, it should be understood that the hemostatic compound may be a bioactive glass material, a mesoporous material, a clay or a combination of any of the foregoing materials with or without a molecular sieve material. In any embodiment, the material is one in which the ratio of the volume to the surface area is very large. For example, typically a volume of material of about one teaspoon provides a surface area of about 50,000 square feet. Also, the hemostatic compound is a material having a positively charged surface.

The molecular sieve particles can be incorporated into the web structure during formation of the web, or they can be impregnated into the finished web by conventional impregnation methods such as, but not limited to, rolling. Various impregnation methods will produce differing loading levels for the molecular sieve materials and also differing degrees of bonding of the molecular sieve particle into the web.

Polymer substrates can be used for the web structures. Solid matrices are useful where the molecular sieve particles reside bound to the surface of the polymer sheet, but open-cell foam having porosity throughout the substrate to form a sponge structure is also desirable in some applications. The term "open-cell" as used herein shall be construed to mean that blood can pass into the cells to contact molecular sieve material resident inside the cell. Polyethylene solid or open-cell sponge material can form such a substrate wherein the molecular sieve particles are bound in place but still allow intimate contact with the blood for desired clotting without leaving molecular sieve particles on the wound surface when the desired degree of clotting is achieved.

Non-woven natural and synthetic cloth substrates like Tyvek® and Gortex® can also be employed. Such substrates allow the underlying skin or tissue to "breathe," thereby providing for longer contact with damaged tissue since gaseous exchange can still take place.

Natural and synthetic substrates can tolerate the dehydration temperatures used to assure that the molecular sieve material has the desired level of water present to control any exothermic reaction and the temperature associated therewith. The dehydration temperature can be as low as 200 degrees C. Higher temperatures up to 400 degrees C reduce the time required to dehydrate the zeolite. However, the present invention is not limited in this regard as other temperatures and dehydration methods known to those skilled in the pertinent art to which the present invention pertains can be employed without departing from the broader aspects of the present invention.

Other substrates having particular utility in the present invention include paper (e.g., adsorbent media paper, polyethylene sheet paper, cellulosic paper, surgical grade kraft paper or Tyvek® artificial paper). Also, synthetic polymeric plastics having particular utility include Mylar® (polyethylene terephalate polyesters), polyethylene film, polypropylene film, polyethylene-polyamide laminated film, polyethylene-polyester laminated film, polypropylene-polyester laminated film, polyethylene-cellophane laminated film, polyethylene-stretched polypropylene laminated film, and combinations of the foregoing. Flexible, air permeable, high temperature resistant, bacteria-impermeable substrate material can also be made of non-woven polyester layers (polymeric fibrous materials such as polypropylene or Reemay® polyester or Veratec® polyester) on either side of and bonded to a microporous membrane. The microporous membrane may be a hydrophobic fluoropolymer membrane such as microporous polytetrafluoroethylene, polyvinylfluoride, polyvinylidenefluoride, polychlorotrifluoroethylene, polyfluoroethylenepropylene, perfluoroalkoxyethylene and tetrafluoroethylene (TFE) copolymers, chlorotrifluoroethylene and ethylene copolymers, TFE and ethylene copolymers, and combinations of the foregoing. However, the present invention is not limited in this regard.

Substrates may also be hydrophilic macromolecules that are capable of swelling when exposed to moisture. Examples of such hydrophilic macromolecules include, but are not limited to, pulp, cellulose, regenerated cellulose (e.g., cellophane, cellulose beads, rayon, cellulose sponge, and the like), cotton, bacterial cellulose, chemically modified cellulose derivatives (e.g., ethyl cellulose, hydroxyethylcellulose, hydroxypropylcellulose, methylcellulose, ethylhydroxyethylcellulose, carboxymethylcellulose, and the like), silk, wool, polyvinyl alcohol, cross-linked polyvinyl alcohol, chitin, chitosan, ethylene-vinyl acetate copolymer, polyvinyls, and water-absorbable macromolecular gels such as polyacrylamides, and the like, as well as combinations of any of the foregoing materials. Hydrophilic macromolecules are disclosed in U.S. Patent No. 5,981,052 and in U.S. Patent No. 6,372,333, both of which are incorporated herein by reference in their entireties.

The molecular sieve material used in the present invention is preferably a zeolite. Other molecular sieve materials or materials having similar properties that may be used include, but are not limited to, faujasite, hydrotalcite, hydroxyapatite, clay minerals, and the like. One exemplary zeolite is a porous aluminosilicate having a structural framework built up from tetrahedral units of SiO₄ and AlO₄ (oxides) linked together via shared oxygen atoms. Each aluminum atom positioned in the framework induces a negative charge. At least one cationic species is interspersed throughout and coulombically bound to the oxide framework to counterbalance the negative charges of the aluminum atoms. An excess of cations located in and coulombically bound to the framework provides a positive charge to the zeolite.

The cationic species included in the framework may be, for example, calcium and sodium moieties. The cations are loaded into the oxide framework and coulombically bound thereto via an ion-exchange process. The ion-exchange is typically effected with an aqueous solution of alkali-, alkaline earth-, and/or transition metal cations to result in a specific ion formulation. The aqueous solutions of the cations are typically lithium chloride, sodium chloride, potassium chloride, strontium nitrate, barium nitrate, ammonium chloride, or silver nitrate.

Typically, the preferred zeolite is a powdered, friable material that is less than about 75% by weight silicon oxide, and preferably less than about 65% by weight silicon oxide; less than about 50% by weight aluminum oxide, and preferably less than about 40% by weight aluminum oxide; less than about 30% by weight sodium oxide, and preferably less than about 20% by weight of sodium oxide; less than about 30% by weight of calcium oxide, and preferably less than about 20% by weight of calcium oxide. The calcium portion contains crystals that are about 5 angstroms in size, and the sodium portion contains crystals that are about 4 angstroms in size. The calcium oxide and sodium oxide may also be replaced by or complemented by the addition of barium oxide, strontium oxide, or lithium oxide as well.

The zeolite may be mixed with or otherwise used in conjunction with other materials having the ability to be dehydrated without significant changes in crystalline structure. Such materials include, but are not limited to, magnesium sulfate, sodium metaphosphate, calcium chloride, dextrin, polysaccharides, combinations of the foregoing materials, and hydrates of the foregoing materials.

The preferred molecular structure of the zeolite is referred to as an "A-type"crystal, namely, one having a cubic crystalline structure that defines round or substantially round openings. Particularly useful forms are described as 5A, 4A, and 3A types. As used herein, the term "A-type crystal" is intended to indicate a crystal having a cubic structure and round holes. Suitable zeolites for use in the applications disclosed herein are also preferably mesoporous and more preferably nanoporous so as to provide increased surface areas. As used herein, the term "nanoporous" is intended to indicate an average pore diameter of about 3 angstroms to about 5 angstroms. Also, other pore sizes can be employed without departing from the broader aspects of the present invention.

A silicon-based zeolite such as that indicated as 4A-type (namely, Na₁₂Si₁₂Al₁₂O₄₈*27H₂O) is particularly useful as the zeolite in the present invention because it can be easily synthesized. Other silicon-based zeolites and materials may be used, particularly those exemplified by sodium metasilicate, potassium metasilicate, potassium orthosilicate, water glass, silica sol, combinations of the foregoing, and the like.

Another zeolite material found to be particularly useful in practicing the present invention is Molsiv 5A, manufactured by UOP, LLC of Des Plaines, IL. Other molecular sieve materials and zeolites can be substituted without departing from the broader aspects of the present invention.

When a cellulose or cellulose-based substrate is used to provide a blood clotting apparatus of the present invention, a zeolite-cellulose composite can be produced by impregnating the substrate with an aqueous solution of the zeolite. The substrate may be immersed in the aqueous solution, or an aqueous solution may be sprayed on the substrate or applied using a coating device. The invention is not limited in this regard, however, as other techniques for imparting zeolite onto the surface of a substrate are within the scope of the present invention.

One exemplary alternate technique for producing a zeolite-cellulose composite includes impregnating a cellulose substrate with an aqueous solution of an aluminum compound followed by immersing the aluminum-impregnated cellulose substrate in an aqueous solution of a silicon compound and a basic substance. In the alternative, a cellulose substrate can first be impregnated with an aqueous solution of a basic substance and then immersed in one of a silicon compound and an aluminum compound, followed by immersion of the base-impregnated substrate in the other of the silicon compound and the aluminum compound. Basic substances that may be used include, but are not limited to, sodium hydroxide, potassium hydroxide, and the like.

Various materials may be mixed with, associated with, or incorporated into the zeolites and/or the substrates to maintain an antiseptic environment at the wound site or to provide functions that are supplemental to the blood clotting functions of the zeolites. Exemplary materials that can be used include, but are not limited to, pharmaceutically-active compositions such as antibiotics, antifungal agents, antimicrobial agents, anti-inflammatory agents, analgesics, antihistamines (e.g., cimetidine, chloropheniramine maleate, diphenhydramine hydrochloride, and promethazine hydrochloride), bacteriostatics, compounds containing silver ions, and the like. Other materials that can be incorporated to provide additional hemostatic functions include ascorbic acid, tranexamic acid, rutin, and thrombin. Botanical agents having desirable effects on the wound site may also be added.

Other materials that may be mixed with, associated with, or incorporated into the zeolites include metal ions. By permeating the zeolite-incorporated substrate with an aqueous solution of a metal salt, metal ions can be incorporated into the zeolite and/or the substrate to provide a catalytic function. Metal ions that may be used include, but are not limited to, silver, copper, zinc, iron, nickel, cobalt, palladium, platinum, combinations of the foregoing, and the like. When silver, copper, or zinc are incorporated into the zeolites or the substrates, antibacterial properties are realized.

Various materials may be mixed with, associated with, adhered to, or incorporated into the zeolites and/or the substrates to improve the strength of the zeolite-laden substrate. Such materials, which are hereinafter referred to as "function improvers," are carriers capable of solidifying after being combined with the zeolite/substrate. Examples of carriers that can be used as function improvers include, but are not limited to, natural resins (e.g., pine resin, shellac, wax, collagen, propolis, urushi, wood powder, and the like), synthetic resins (e.g., polyolefins, polyamides, polyvinyl alcohols, phenols, and the like), rubbers, calcium carbonate, calcium silicate, titanium dioxide, zeolite, clay, colloidal silica, apatite-based compounds, talsite-based compounds, combinations of the foregoing, and the like.

The function improvers may also be in the form of fibers. Such fibers include, but are not limited to, polyester fibers (such as polyethylene terephthalate), polyolefin fibers, polyurethane fibers, polyacrylic fibers, cellulose fibers (such as rayon and the like), natural fibers (such as wool, silk, cotton, hemp, kenaf, and the like), synthetic fibers (such as glass, carbon, and metal), charcoal fibers, and combinations of the foregoing. When in fibrous form, the function improver may be knitted, woven, non-woven, or felted. Preferably, when the fibers are arranged into their particular form, the resulting material is bibulous in nature.

The control of the moisture content of the zeolite in the substrate is related to its effectiveness. Preferred moisture content is between about 5 and 25% by weight, more preferred between about 7 and about 19 weight % and most preferred between 10 and 15 weight %. In such a composition, the moisture content of the zeolite can be adjusted by drying and then re-hydrating, or a combination of drying and re-hydrating such that the zeolite has the desired specific moisture content. Alternatively, the composition may be fully saturated with water and subsequently dried to a specific water content. In the drying of the zeolite, the bound water is removed to allow the crystalline structure of the zeolite to remain intact. In the re-hydration of the zeolite, the most active adsorption sites are hydrated first and then less active sites are hydrated. As the zeolite's degree of hydration increases, the heat of hydration decreases. More specifically, when the composition is applied to the blood, water in the blood is adsorbed by the zeolite. Upon adsorption of this water, heat is generated. At higher levels of hydration (hydration of the zeolite prior to its application to blood), less heat is generated when the composition is applied to blood. Thus, when the composition is applied to blood directly at a wound site, the amount of heat transferred to the tissue surrounding the wound site is reduced.

According to another aspect of the present invention, a method of forming a blood-clotting composition comprises the steps of providing a zeolite in hydrated form embedded in or otherwise attached to a substrate and adjusting the moisture content of the zeolite to a specific moisture content such that upon application of the composition to a wound, the heat of hydration is reduced and the amount of heat transferred to the bleed site is reduced.

The substrates impregnated with molecular sieve materials, preferably zeolite, can be used in a wide variety of medical applications. They may be used as a topical bandage where the impregnated substrate forms the portion of the bandage that contacts the bleeding wound area and an adhesive outer periphery keeps it in contact with the wound. It may also be used in various dental procedures where it is desired to minimize bleeding at the procedure site. Veterinary applications for animals parallel the human medical applications. In the surgical uses described below, this form offers the benefit of the bandage form of assuring that it stays in contact with the wound or incision area though the use of either adhesive areas around the clotting portion of the bandage or the zeolite impregnated substrate may of course be overwrapped with a strap or dressing to keep it in contact with the desired area.

The devices of the present invention can also be used in surgical procedures where it is desired to quickly and efficiently stop undesired bleeding by clotting blood flowing from a wound being repaired or from an incision. The present invention is particularly useful in difficult internal organ repairs such as the liver, spleen, and kidney tissues where stopping blood flow can be problematic particularly in situations involving large surface area damage to those organs. The devices of the present invention have been found to be very effective in stopping blood flow through efficient clotting mechanisms. In this use the zeolite impregnated substrate is applied to the area exhibiting bleeding for a time sufficient to induce clotting. The clotted area repairs itself in a most surprising manner resulting in good clinical results in liver and spleen trauma situations in mammals.

Neurosurgical procedures also can benefit from use of the large area clotting abilities of the impregnated substrate of the present invention to stop hemorrhaging of brain tissue during intracranial procedures.

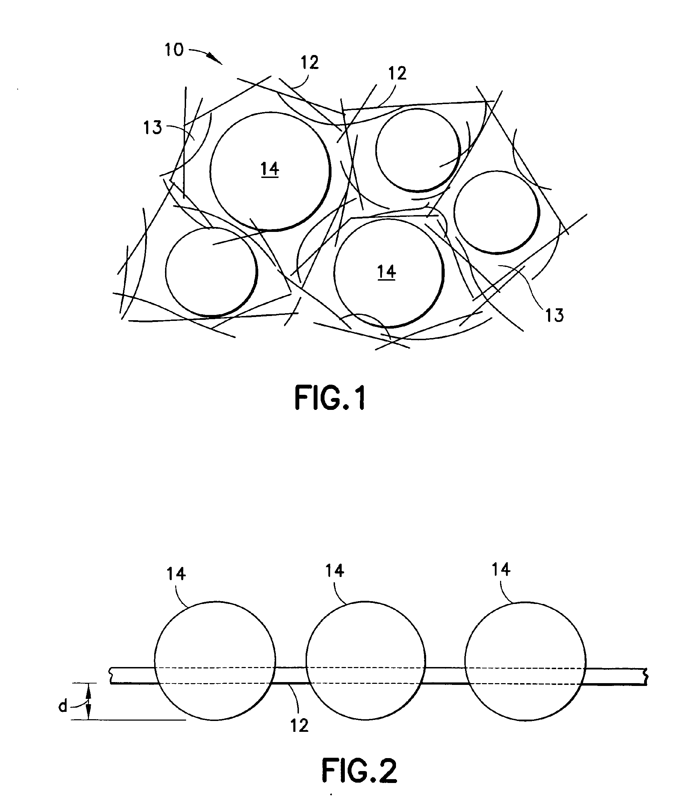

As shown in FIG. 1, an apparatus for promoting the clotting of blood is generally designated by the reference number 10 and comprises a substrate 12 composed of paper or cloth and zeolite particles 14 retained thereon by impregnation into interstices 13 defined by the substrate 12. As illustrated, the substrate 12 is shown as being planar, and only a few zeolite particles 14 are shown for illustration purposes. While the zeolite particles have been shown and described as being retained in interstices defined by the substrate, the present invention is not limited in this regard, as the zeolite particles can be adhesively or otherwise bonded to the substrate without departing from the broader aspects of the present invention.

The zeolite particles in the preferred embodiment of the present invention exhibit a cubic structure and have a median particle size of approximately 7 microns. The particles define pore sizes preferably between about 4.5 to 5 angstroms. However, the present invention is not limited in this regard as other pore sizes known to those skilled in the pertinent art to which the present invention pertains can be substituted without departing from the broader aspects of the present invention. Moreover, while cubic structures and particle sizes of approximately 7 microns have been described, the present invention is not limited in this regard as other structures such as spherical or irregular and differing particular sizes can also be substituted.

In any embodiment (balls, beads, pellets, etc.), less particle surface area is available for contact with blood as particle size increases. Therefore, the rate of clotting can be controlled by varying the particle size. Furthermore, the adsorption of moisture (which also has an effect on the exothermic effects of the zeolite) can also be controlled.

Referring now to FIG. 2, the substrate 12 defines a plurality of interstices therein to retain the zeolite particles 14 but still allow flowing blood to contact the zeolite. Because the substrate 12 may partially surround the zeolite particles 14, a portion of the particles may extend through the interstices by a distance d. This allows the zeolite to directly contact tissue and blood to which the clotting device is applied. Accordingly, blood emanating from the tissue contacts the zeolite particles 14, and the liquid phase thereof is wicked into the zeolite material, thereby facilitating the clotting of the blood. However, it is not a requirement of the present invention that the zeolite particles protrude through the substrate.

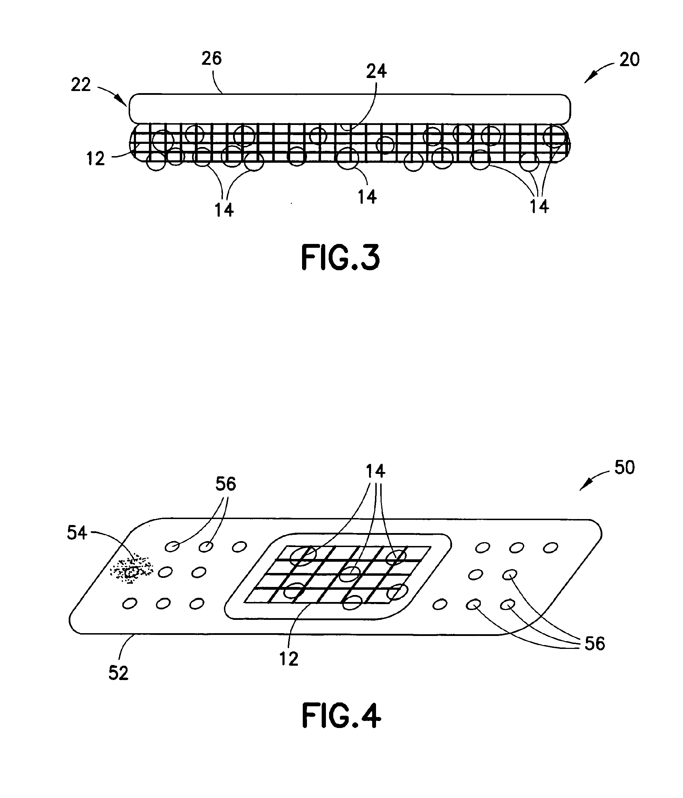

As shown in FIG. 3, a pad 20 comprises the substrate 12, zeolite (or other molecular sieve) particles 14 retained thereon, and a support 22 to which pressure may be applied in the application of the pad 20 to a bleed site.

The substrate 12 is stitched, glued, clamped, or otherwise mounted to the support 22. The support 22 comprises an undersurface 24 against which the zeolite particles 14 are held by the substrate 12 and a top surface 26. The undersurface 24 is substantially impermeable to the zeolite particles 14 (migration of the particles into the support 22 is prevented) and is further resistant to the absorption of water or other fluids. The top surface 26 is capable of having pressure exerted thereon by a person applying the pad 20 to a bleeding wound or by a weight supported on the top surface 26. The entire support 22 is rigid or semi-rigid so as to allow the application of pressure while minimizing discomfort to the patient.

To apply the pad 20 to a bleeding wound, the pad 20 is removed from its packaging and placed on the bleeding wound. The zeolite particles 14 are either in direct contact with the tissue of the wound or are in direct contact with the blood. Pressure may be applied to the wound by pressing on the top surface 26 with a hand or by placing a weight on the surface, thereby facilitating the contact between the zeolite particles 14 and the wound and promoting the adsorption of the liquid phase of the blood. The pad 20 (with or without a weight) may also be held onto the wound using a strapping device such as a belt, an elastic device, hook-and-loop material, combinations of the foregoing devices and materials, and the like.

Referring now to FIG. 4, a bandage, shown at 50, includes zeolite particles 14 (or some other molecular sieve material) retained on the substrate 12, the substrate being mounted to the flexible web 52 that can be applied to a wound (for example, using a pressure-sensitive adhesive to adhere the bandage 50 to the skin of a wearer). The substrate 12 is stitched, glued, or otherwise mounted to a flexible web 52 to form the bandage 50.

The flexible web 52 is a plastic, paper, or a cloth member that is conducive to being retained on the skin of an injured person or animal on or proximate a bleeding wound. An adhesive 54 is disposed on a surface of the flexible web 52 that engages the skin of the injured person or animal. Particularly if the flexible web 52 is a non-breathable plastic material, the flexible web may include holes 56 to allow for the dissipation of moisture evaporating from the skin surface. The flexible web 52 can also extend outwardly from the substrate 12 an amount sufficient to allow it to be tied around a portion of a person's body. As such the web can be made of cloth, gauze, or other suitable material.

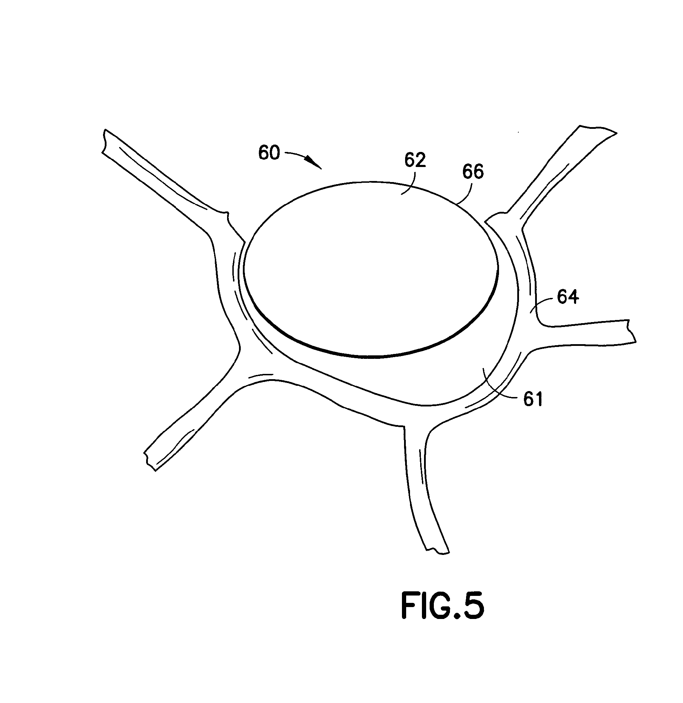

As shown in FIG. 5 a single cell 61 of an open-cell foam substrate 60 is shown having a zeolite particle 62 embedded in the pore. The wall 64 of the cell 61 (which is also sometimes called an interstice of a foam structure) has an opening 66 therein which allows blood to flow into contact with the zeolite particle embedded in the cell. This direct contact of the zeolite with the blood fluids allows for proper adsorption of water from the blood to initiate clotting of the solid portions of the blood.

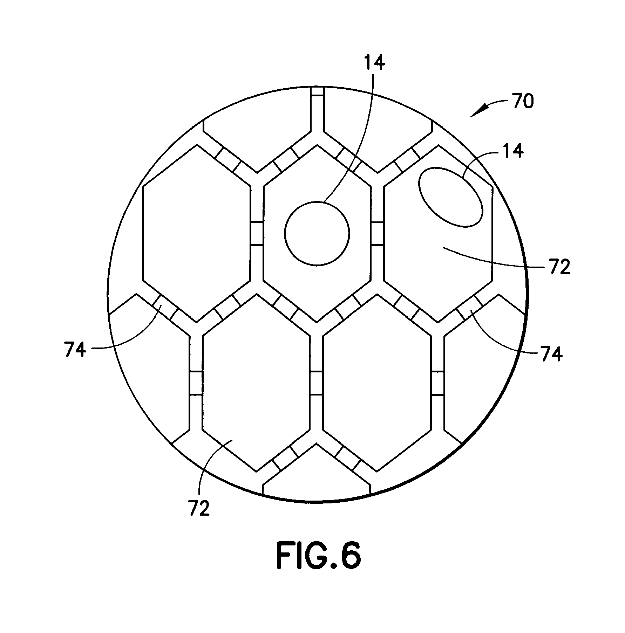

FIG. 6 shows substrate 70 having honeycomb shaped cells 72 in the foam substrate with breaks 74 in cell walls which allows the water from the blood to contact the zeolite particles 14 shown in some of the cells.

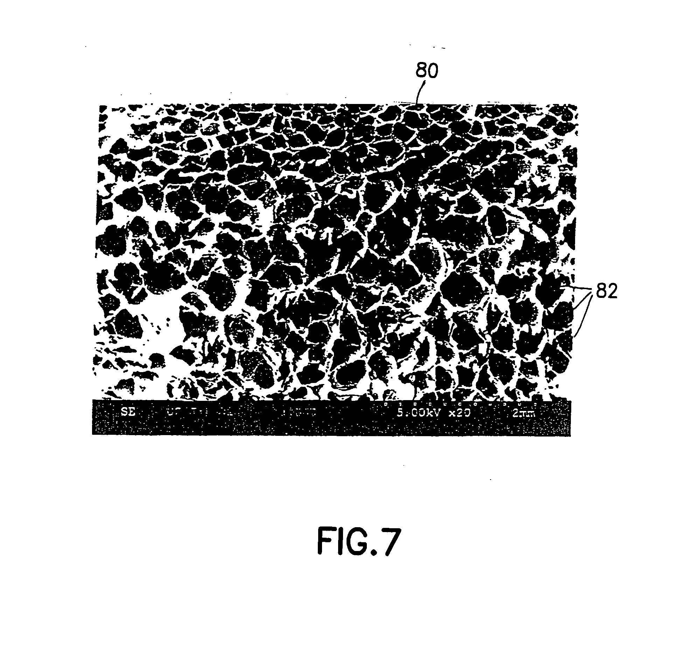

FIG. 7 is a photomicrograph 80 of a section of a polymeric open cell foam matrix suitable for inclusion of zeolite particles (not shown) in the cell structure. It has millions of small interstices 82 into which the molecular sieve material such as zeolite is imbedded.

In the preparation of zeolite material for the devices of the present invention (i.e., formation of the sieve material into sponge form), an initial level of hydration of the zeolite may be controlled by the application of heat to the zeolite material either before or after the material is incorporated into the substrate.

Although this invention has been shown and described with respect to the detailed embodiments thereof, it will be understood by those of skill in the art that various changes may be made and equivalents may be substituted for elements thereof without departing from the scope of the invention. In addition, modifications may be made to adapt a particular situation or material to the teachings of the invention without departing from the essential scope thereof. Therefore, it is intended that the invention not be limited to the particular embodiments disclosed in the above detailed description, but that the invention will include all embodiments falling within the scope of the appended claims.

## Claims

1. An apparatus (10) for promoting the clotting of blood, said apparatus (10) being **characterized by** a substrate (12) and a zeolite (14) in a powder form deposited on said substrate (12), said zeolite (14) having a matrix of porous aluminosilicate, and at least one cationic species interspersed throughout and coulombically bound to said matrix, said cationic species providing a positive charge to said matrix.

2. The apparatus (10) of claim 1, wherein said zeolite (14) includes:
less than about 75% by weight of silicon oxide;
less than about 50% by weight of aluminum oxide;
less than about 30% by weight of sodium oxide; and
less than about 30% by weight of calcium oxide;
wherein upon application of said substrate(12) having said zeolite (14) deposited thereon to a bleed site, said zeolite (14) causes blood at said bleed site to clot.

3. The apparatus (10) of claim 1, wherein said substrate (12) is selected from the group consisting of adsorbent media paper and polyethylene sheet paper.

4. The apparatus (10) of claim 2, wherein said silicon oxide is less than about 65% by weight, said aluminum oxide is less than about 40% by weight, said sodium oxide is less than about 20% by weight, and said calcium oxide is less than about 20% by weight.

5. The apparatus (10) of claim 1, wherein said apparatus (10) includes an adhesive (54) on said substrate (12) having said zeolite (14) thereon, said adhesive (54) for adhesively and removably attaching said substrate (12) having said zeolite (14) thereon to skin.

6. The apparatus (10) of claim 1, wherein said substrate (12) is attached to a flexible carrier (52) having portions extending outwardly from said substrate (12) so as to form a bandage (50) that can be wrapped around a portion of a body with said zeolite (14) contacting a bleed site.

7. The apparatus (10) of claim 1, wherein said substrate (12) is flexible.

8. The apparatus (10) of claim 1, wherein said zeolite (14) is a powder having a median particle size of about seven microns.

9. The apparatus (10) of claim 1, wherein said substrate (12) defines interstices (13) therein and said zeolite (14) is embedded in interstices (13) of said substrate (12).

10. The apparatus (10) of claim 9, wherein at least a portion of said zeolite (14) protrudes outwardly from at least a portion of said interstices (13) so that said portion of said zeolite (14) directly contacts blood at a wound site.

11. The apparatus (10) of claim 10, wherein said substrate (12) is coupled to a substantially impermeable material layer (24) to allow said zeolite (14) to be brought into contact with and compressed against a bleed site.

12. The apparatus (10) of claim 1, wherein said apparatus (10) includes at least one of an antimicrobial agent, an anti-inflammatory agent, an analgesic, an antihistamine, and a compound containing silver ions attached to said substrate (12).

13. The apparatus (10) of claim 1, wherein said substrate (12) is selected from the group consisting of polymer matrices, natural cloth, synthetic cloth, non-woven natural cloth, and non-woven synthetic cloth.

14. The apparatus (10) of claim 1, wherein said substrate (12) is a synthetic polymeric matrix selected from the group consisting of polyethylene terephalate polyesters, polyethylene film, polypropylene film, polyethylene-polyamide laminated film, polyethylene-polyester laminated film, polypropylene-polyester laminated film, polyethylene-cellophane laminated film, and polyethylene-stretched polypropylene laminated film.

15. The apparatus (10) of claim 1, wherein said substrate (12) is selected from the group consisting of cellulosic paper, kraft paper and artificial paper.

16. The apparatus (10) of claim 1, wherein said substrate (12) is a synthetic polymeric matrix having open-cell foam porosity (66) in at least a portion thereof.

17. An agent (10) for promoting the clotting of blood, said agent (10) being **characterized by** a porous web structure (12); and a compound (14) capable of providing hemostasis incorporated into said porous web structure (12); wherein said compound (14) capable of providing hemostasis includes at least one cationic species interspersed throughout and coulombically bound to said web structure (12), said cationic species providing a positive charge to said web structure (12).

18. The agent (10) of claim 17, wherein a material from which said porous web structure (12) is fabricated is selected from the group consisting of paper, polymeric material, natural cloth, synthetic cloth, hydrophilic macromolecules, and combinations of the foregoing.

19. The agent (10) of claim 18, wherein said paper is selected from the group consisting of fibrous paper, cellulosic paper, kraft paper, artificial paper, and combinations of the foregoing.

20. The agent (10) of claim 18, wherein said polymeric material is selected from the group consisting of open-cell foam, polyethylene solid sponge material, polymeric plastics, and combinations of the foregoing.

21. The agent (10) of claim 20, wherein said polymeric plastics are selected from the group consisting of polyethylene terephthalate polyesters, polyethylene, polypropylene, polyethylene-polyamide laminates, polyethylene-cellophane laminates, polyethylene-stretched polypropylene laminates, and combinations of the foregoing.

22. The agent (10) of claim 20, wherein said polymer plastics are polyesters bonded to at least one hydrophobic fluoropolymer membrane.

23. The agent (10) of claim 22, wherein said at least one hydrophobic fluoropolymer membrane is selected from the group consisting of polytetrafluoroethylene, polyvinylfluoride, polyvinylidenefluoride, polychlorotrifluoroethylene, polyfluoroethylenepropylene, perfluoroalkoxyethylene and tetrafluoroethylene (TFE) copolymers, chlorotrifluoroethylene and ethylene copolymers, TFE and ethylene copolymers, and combinations of the foregoing.

24. The agent (10) of claim 18, wherein said hydrophilic macromolecules are selected from the group consisting of pulp, cellulose, regenerated cellulose, cotton, bacterial cellulose, chemically modified cellulose derivatives, silk, wool, polyvinyl alcohol, cross-linked polyvinyl alcohol, chitin, chitosan, ethylene-vinyl acetate copolymer, polyvinyls, water-absorbable macromolecular gels, and combinations of the foregoing.

25. The agent (10) of claim 24, wherein said regenerated cellulose is selected from the group consisting of cellophane, cellulose beads, rayon, cellulose sponge, and combinations of the foregoing.

26. The agent (10) of claim 24, wherein said chemically modified cellulose derivatives are selected from the group consisting of ethyl cellulose, hydroxyethylcellulose, hydroxypropylcellulose, methylcellulose, ethylhydroxyethylcellulose, carboxymethylcellulose, and combinations of the foregoing.

27. The agent (10) of claim 17, wherein said compound capable of providing hemostasis is selected from the group consisting of molecular sieve materials, bioactive glass materials, mesoporous materials, clays, faujasite, hydrotalcite, hydroxyapatite, and combinations of the foregoing.

28. The agent (10) of claim 27, wherein said molecular sieve material is a zeolite (14).

29. The agent (10) of claim 28, wherein said zeolite (14) includes:
less than about 75% by weight of silicon oxide;
less than about 50% by weight of aluminum oxide;
less than about 30% by weight of sodium oxide; and
less than about 30% by weight of calcium oxide.

30. The agent (10) of claim 28, wherein said zeolite (14) includes an aluminosilicate having a structural framework of SiO₄ and AlO₄ linked together via shared oxygen atoms.

31. The agent (10) of claim 28, wherein said cationic species is selected from the group consisting of sodium moieties and calcium moieties.

32. The agent (10) claim 31, wherein said positive charge is effected by an excess of at least one of said sodium moieties and said calcium moieties.

33. The agent (10) of claim 28, wherein said zeolite (14) is silicon-based.

34. The agent (10) of claim 33, wherein said silicon-based zeolite (14) is selected from the group consisting of sodium metasilicate, potassium metasilicate, potassium orthosilicate, water glass, silica sol, and combinations of the foregoing.

35. The agent (10) of claim 28, wherein a metal ion is incorporated into said zeolite (14), said metal ion selected from the group consisting of silver, copper, zinc, iron, nickel, palladium, platinum, and combinations of the foregoing.

36. The agent (10) of claim 28, including a material selected from the group consisting of natural resins, synthetic resins, rubbers, calcium carbonate, calcium silicate, titanium dioxide, clay, colloidal silicate, apatite-based compounds, talsite-based compounds, and combinations of the foregoing.

37. The agent (10) of claim 28, including a material selected from the group consisting of polyester fibers, polyolefin fibers, polyurethane fibers, polyacrylic fibers, cellulose fibers, natural fibers, synthetic fibers, charcoal fibers, and combinations of the foregoing.

38. The agent (10) of claim 28, including a pharmaceutically-active composition selected from the group consisting of antibiotics, antifungal agents, anti-inflammatory agents, analgesics, antihistamines, bacteriostatics, compounds containing silver ions, ascorbic acid, tranexamic acid, rutin, thrombin, botanical agents, and combinations of the foregoing.

39. A method of fabricating a blood clotting apparatus (10), said method being **characterized by** the steps of:
providing a cellulose-based substrate (12);
providing a zeolite (14);
incorporating at least one cationic species into a structure of said zeolite (14) to impart a positive charge to said zeolite (14); and
impregnating said cellulose-based substrate (12) with said zeolite (14).

40. The method of claim 39, wherein said step of incorporating at least one cationic species into said zeolite (14) includes ion-exchanging said zeolite (14) with an aqueous solution having at least one of an alkali-, an alkaline earth-, and a transition metal.

41. The method of claim 39, wherein said step of impregnating said cellulose-based substrate (12) with said zeolite (14) includes,
immersing said cellulose-based substrate (12) into an aqueous solution of said zeolite (14), and
immersing said cellulose-based substrate (12) into an aqueous solution of a basic substance.

42. The method of claim 41, wherein said step of immersing said cellulose-based substrate (12) into said aqueous solution of said zeolite (14) includes,
immersing said cellulose-based substrate (12) into an aqueous solution of a silicon compound, and
immersing said cellulose-based substrate (12) into an aqueous solution of an aluminum compound.
